# EUROPÄISCHE PATENTSCHRIFT

(11) **EP 1 315 718 B1**
(45) Veröffentlichungstag und Bekanntmachung des Hinweises auf die Patenterteilung: **28.11.2007**
(21) Anmeldenummer: 01976107.1
(22) Anmeldetag: 18.08.2001
(51) Int. Cl.: C07D 405/12, A61K 31/517

(54) **BICYCLISCHE HETEROCYCLEN, DIESE VERBINDUNGEN ENTHALTENDE ARZNEIMITTEL, DEREN VERWENDUNG UND VERFAHREN ZU IHRER HERSTELLUNG**
BICYCLIC HETEROCYCLES, MEDICAMENTS CONTAINING THESE COMPOUNDS, THEIR USE, AND METHODS FOR THE PRODUCTION THEREOF
HETEROCYCLES BICYCLIQUES, MEDICAMENTS CONTENANT LESDITS COMPOSES, LEUR UTILISATION ET PROCEDES PERMETTANT DE LES PRODUIRE

(30) Priorität: 26.08.2000 DE 10042059
(43) Veröffentlichungstag der Anmeldung: 04.06.2003
(73) Patentinhaber: Boehringer Ingelheim Pharma GmbH & Co. KG, 55216 Ingelheim am Rhein (DE)
(72) Erfinder: HIMMELSBACH, Frank, 88441 Mittelbiberach (DE); LANGKOPF, Elke, 88447 Warthausen (DE); JUNG, Birgit, 55270 Schwabenheim (DE); BLECH, Stefan, 88447 Warthausen (DE); SOLCA, Flavio, A-1230 Vien (AT)
(86) Internationale Anmeldenummer: PCT/EP2001/009533
(87) Internationale Veröffentlichungsnummer: WO 2002/018372

(56) Entgegenhaltungen:
- EP-A- 0 566 226
- WO-A-00/18740
- WO-A-00/55141
- WO-A-96/33980

## Beschreibung

Gegenstand der vorliegenden Erfindung sind bicyclische Heterocyclen der allgemeinen Formel 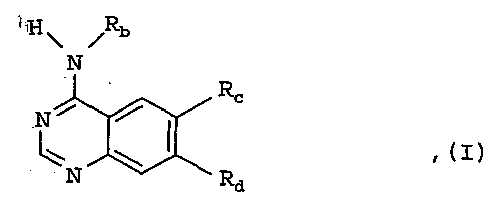 deren Tautomeren, deren Stereoisomere und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen, von Erkrankungen der Lunge und der Atemwege und deren Herstellung. WO 0055141, EP 0566226, WO 96 33 980 sowie WO 0018740 beschreiben Chinazolin und Chinocine als Tyrosinkinase Inbibitoren.

In der obigen allgemeinen Formel I bedeutet
R_{b} eine 1-Phenylethyl-, 3-Methylphenyl-, 3-Chlorphenyl-, 3-Bromphenyl- oder 3-Chlor-4-fluorphenylgruppe,
R_{c} eine -A-B Gruppe, in der
A eine -OCH₂CH₂-, -OCH₂CH₂CH₂- oder -OCH₂CH₂CH₂CH₂-Gruppe, wobei der Alkylenteil jeweils mit dem Rest B verknüpft ist, und B eine
eine Piperidinogruppe, die in 4-Stellung durch eine 2-Oxo-morpholino- oder 2-Oxo-morpholinomethylgruppe substituiert ist, wobei der 2-Oxo-morpholinoteil jeweils durch eine oder zwei Methylgruppen substituiert sein kann,
und R_{d} eine Methoxy-, Cyclopropylmethoxy-, Tetrahydrofuran-3-yloxy-, Tetrahydropyran-3-yloxy-, Tetrahydropyran-4-yloxy-, Tetrahydrofuranylmethoxy- oder Tetrahydropyranylmethoxygruppe bedeuten,
deren Tautomere, Stereoisomere und deren Salze.

Die Verbindungen der allgemeinen Formel I lassen sich beispielsweise nach folgenden Verfahren herstellen:

Umsetzung einer gegebenenfalls im Reaktionsgemisch gebildeten Verbindung der allgemeinen Formel 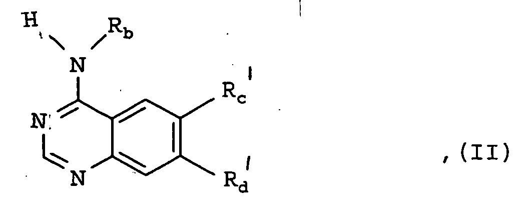 in der
R_{b} wie eingangs erwähnt definiert ist,
einer der Reste R_{c}' oder R_{d}' eine für R_{c} oder R_{d} eingangs erwähnte -C-D Gruppe und
der andere der Reste R_{c}' oder R_{d}' eine -A'-Z₁ Gruppe bedeuten, wobei
   A' eine -O-C₁₋₆-alkylengruppe und
   Z₁ eine austauschbare Gruppe wie ein Halogenatom oder eine substituierte Sulfinyl- oder Sulfonylgruppe, z. B. ein Chlor- oder Bromatom, eine Methylsulfinyl-, Propylsulfinyl-, Phenylsulfinyl-, Benzylsulfinyl-, Methylsulfonyl-, Propylsulfonyl-, Phenylsulfonyl- oder Benzylsulfonylgruppe bedeutet,
mit einer Verbindung der allgemeinen Formel

H - G , (III)

in der
G einen der für B eingangs erwähnten Reste darstellt, der über ein Stickstoffatom mit dem Rest A verknüpft ist.

Die Umsetzung wird zweckmäßigerweise in einem Lösungsmittel wie Acetonitril, Tetrahydrofuran, Dioxan, Toluol, Chlorbenzol, Dimethylformamid, Dimethylsulfoxid, Methylenchlorid, Ethylenglycoldiethylether oder Sulfolan gegebenenfalls in Gegenwart einer anorganischen oder tertiären organischen Base, z.B.

Natriumcarbonat oder Kaliumhydroxid, einer tertiären organischen Base wie Triethylamin oder N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkaliodid bei Temperaturen zwischen -20 und 150°C, vorzugsweise jedoch bei Temperaturen zwischen -10 und 100°C, durchgeführt. Die Umsetzung kann jedoch auch ohne Lösungsmittel oder in einem Überschuß der eingesetzten Verbindung der allgemeinen Formel III durchgeführt werden.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Trityl-, Benzyl- oder Tetrahydropyranylgruppe,
als Schutzreste für eine Carboxygruppe die Trimethylsilyl-, Methyl-, Ethyl-, tert.Butyl-, Benzyl- oder Tetrahydropyranylgruppe und
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure; Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II und III sind teilweise literaturbekannt oder man erhält diese nach an sich literaturbekannten Verfahren (siehe Beispiele I bis XIV).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:

Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Hier wurde eine Interleukin-3-(IL-3) abhängige Zellinie murinen Ursprungs verwendet, die derart genetisch verändert wurde, daß sie funktionellen humanen EGF-R exprimiert. Die Proliferation dieser F/L-HERc genannten Zellen kann daher entweder durch murines IL-3 oder durch EGF stimuliert werden (siehe von Rüden, T. et al. in EMBO J. 7, 2749-2756 (1988) und Pierce, J. H. et al. in Science 239, 628-631 (1988)).

Als Ausgangsmaterial für die F/L-HERc Zellen diente die Zelllinie FDC-P₁, deren Herstellung von Dexter, T. M. et al, in J. Exp. Med. 152, 1036-1047 (1980) beschrieben wurde. Alternativ können aber auch andere Wachstumsfaktor-abhängige Zellen verwendet werden (siehe beispielsweise Pierce, J. H. et al. in Science 239, 628-631 (1988), Shibuya, H. et al. in Cell 70, 57-67 (1992) und Alexander, W. S. et al. in EMBO J. 10, 3683-3691 (1991)). Zur Expression der humanen EGF-R cDNA (siehe Ullrich, A. et al. in Nature 309, 418-425 (1984)) wurden rekombinante Retroviren verwendet, wie in von Rüden, T. et al., EMBO J. 7, 2749-2756 (1988) beschrieben, mit dem Unterschied, daß zur Expression der EGF-R cDNA der retrovirale Vektor LXSN (siehe Miller, A. D. et al. in BioTechniques 7, 980-990 (1989)) eingesetzt wurde und als Verpackungszelle die Linie GP+E86 (siehe Markowitz, D. et al. in J. Virol. 62, 1120-1124 (1988)) diente.

Der Test wurde wie folgt durchgeführt:

F/L-HERc Zellen wurden in RPMI/1640 Medium (BioWhittaker), supplementiert mit 10 % foetalem Rinderserum (FCS, Boehringer Mannheim), 2 mM Glutamin (BioWhittaker), Standardantibiotika und 20 ng/ml humanem EGF (Promega), bei 37°C und 5% CO₂ kultiviert. Zur Untersuchung der inhibitorischen Aktivität der erfindungsgemäßen Verbindungen wurden 1,5 x 10⁴ Zellen pro Vertiefung in Triplikaten in 96-Loch-Platten in obigem Medium (200 µl) kultiviert, wobei die Proliferation der Zellen entweder mit EGF (20 ng/ml) oder murinem IL-3 stimuliert wurde. Als Quelle für IL-3 dienten Kulturüberstände der Zellinie X63/0 mIL-3 (siehe Karasuyama, H. et al.in Eur. J. Immunol. 18, 97-104 (1988)). Die erfindungsgemäßen Verbindungen wurden in 100% Dimethylsulfoxid (DMSO) gelöst und in verschiedenen Verdünnungen den Kulturen zugefügt, wobei die maximale DMSO Konzentration 1% betrug. Die Kulturen wurden für 48 Stunden bei 37°C inkubiert.

Zur Bestimmung der inhibitorischen Aktivität der erfindungsgemäßen Verbindungen wurde die relative Zellzahl mit dem Cell Titer 96™ AQᵤₑₒᵤₛ Non-Radioactive Cell Proliferation Assay (Promega) in O.D. Einheiten gemessen. Die relative Zellzahl wurde in Prozent der Kontrolle (F/LHERc Zellen ohne Inhibitor) berechnet und die Wirkstoffkonzentration, die die Proliferation der Zellen zu 50% hemmt (IC₅₀), abgeleitet. Hierbei wurden folgende Ergebnisse erhalten:

| Verbindung | Hemmung der EGF-abhängigen Proliferation IC₅₀ [nM] |
|---|---|
| (Beispiel Nr.)* | |
| 1 | 4 |
| 3 | 62 |
| 3(1) | 11 |
| 4 | 67 |

| | |
|---|---|
| *Nicht Teil der Erfindung | |

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome,
desweiteren zur Behandlung von Nasenpolypen sowie von Polypen des Gastrointestinaltraktes unterschiedlicher Genese wie z.B. villöse oder adenomatöse Polypen des Dickdarms, aber auch von Polypen bei familiärer Polyposis coli, bei Darmpolypen im Rahmen des Gardner-Syndroms, bei Polypen im gesamten Magen-Darm-Trakt bei Peutz-Jeghers-Syndrom, bei entzündlichen Pseudopolypen, bei juvenilen Polypen, bei Colitis cystica profunda und bei Pneumatosis cystoides intestinales.

Außerdem können die Verbindungen der allgemeinen Formel *I* und deren physiologisch verträglichen Salze zur Behandlung von Nierenerkrankungen, insbesondere bei zystischen Veränderungen wie bei Zystennieren, zur Behandlung von Nierenzysten, die idiopathischer Genese sein können oder im Rahmen von Syndromen auftreten wie z.B. bei der tuberöser Sklerose, bei dem von-Hippel-Lindau-Syndrom, bei der Nephronophthisis und Markschwammniere sowie anderer Krankheiten verwendet werden, die durch aberrante Funktion von Tyrosinkinasen verursacht werden, wie z.B. epidermaler Hyperproliferation (Psoriasis), inflammatorischer Prozesse, Erkrankungen des Immunsystems, Hyperproliferation hämatopoetischer Zellen etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch, broncholytisch und/oder entzündungshemmend wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden oder entzündungshemmenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intrarektal, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0,01-100 mg/kg Körpergewicht, vorzugsweise bei 0,1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/-Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

### Herstellung der Ausgangsverbindungen: (Mit "*" gekennzeichnete Verbindungen/ Verfahren sind nicht Teil der Erfindung)

### Beispiel I*

### 4-[(3-Chlor-4-fluor-phenyl)amino]-6-cyclopentyloxy-7-[2-(piperazin-1-yl)-ethoxy]-chinazolin

Zu 740 mg 4-[(3-Chlor-4-fluor-phenyl)amino]-6-cyclopentyloxy-7-{2-[4-(tert.butyloxycarbonyl)-piperazin-1-yl]-ethoxy}-chinazolin in 10 ml Methylenchlorid werden 2.00 ml Trifluoressigsäure getropft. Die Reaktionslösung wird über Nacht bei Raumtemperatur gerührt. Zur Aufarbeitung wird das Reaktionsgemisch eingeengt, mit 20 ml Wasser versetzt und mit konzentrierter wäßriger Ammoniaklösung alkalisch gestellt. Die wässrige Phase wird mit Essigester extrahiert. Die vereinigten Extrakte werden mit gesättigter Natriumcarbonat-Lösung und gesättigter Natriumchlorid-Lösung gewaschen, über Magnesiumsulfat getrocknet und eingeengt. Es bleibt ein hellgelber Feststoff zurück.
Ausbeute: 570 mg (93 % der Theorie),
Schmelzpunkt: 134-137,5°C
Massenspektrum (ESI⁻): m/z = 484, 486 [M-H]⁻

Analog Beispiel I werden folgende Verbindungen erhalten:
(1) 4-[(3-Chlor-4-fluor-phenyl)amino]-6-cyclopentylmethoxy-7-[2-(piperazin-1-yl)-ethoxy]-chinazolin
   R_{f}-Wert: 0.05 (Kieselgel, Methylenchlorid/Methanol/konzentrierte wäßrige Ammoniaklösung = 90:10:0.1)
   Massenspektrum (ESI⁻) : m/z = 498, 500 [M-H]⁻
(2) Perhydro-pyrazino[2,1-c][1,4]oxazin-3-on x 2 Trifluoressigsäure (Das Reaktionsgemisch wird ohne wäßrige Aufarbeitung eingeengt)
   Massenspektrum (ESI⁺) : m/z = 157 [M+H]⁺
(3) 4-[(3-Chlor-4-fluor-phenyl)amino]-6-cyclopropylmethoxy-7-[2-(piperazin-1-yl)-ethoxy]-chinazolin
   R_{f}-Wert: 0.10 (Kieselgel, Methylenchlorid/Methanol/konzentrierte wäßrige Ammoniaklösung = 90:10:1)
   Massenspektrum (ESI⁺) : m/z = 472, 474 [M+H]⁺
(4) 2-Oxo-3-[(piperidin-4-yl)sulfanyl]-tetrahydrofuran x Trifluoressigsäure
   (Das Reaktionsgemisch wird ohne wäßrige Aufarbeitung eingeengt.)
   R_{f}-Wert: 0.66 (Reversed Phase DC-Fertigplatte (E. Merck), Acetonitril/Wasser/Trifluoressigsäure = 50:50:1)
   Massenspektrum (ESI⁺): m/z = 202 [M+H]⁺

### Beispiel II*

### 4-[(3-Chlor-4-fluor-phenyl)amino]-6-cyclopentyloxy-7-{2-[4-(tert.butyloxycarbonyl)-piperazin-1-yl]-ethoxy}-chinazolin

Zu 940 mg 4-[(3-Chlor-4-fluor-phenyl)amino]-6-cyclopentyloxy-7-(2-brom-ethoxy)-chinazolin und 1.00 g N-(tert.Butyloxycarbonyl)-piperazin in 30 ml Acetonitril werden bei Raumtemperatur 340 mg 1,8-Diazabicyclo[5.4.0]undec-7-en gegeben. Das Reaktionsgemisch wird fünf Stunden auf 60°C erhitzt. Dann werden nochmals 0.2 g N-(tert.Butyloxycarbonyl)-piperazin und etwas 1,8-Diazabicyclo[5.4.0]undec-7-en zugesetzt. Die gelbe Reaktionslösung wird zwei Stunden bei 60°C und anschließend über Nacht bei Raumtemperatur gerührt, wobei sich ein weißer Niederschlag bildet. Dieser wird abgesaugt, mit wenig Acetonitril nachgewaschen und getrocknet. Man erhält 453 mg des gewünschten Produktes als weißen Feststoff. Die Mutterlauge wird eingeengt und der Kolbenrückstand über eine Kieselgelsäule mit Methylenchlorid/Methanol (95:5) chromatographiert. Es werden nochmals 300 mg des gewünschten Produktes erhalten. Ausbeute: 753 mg (66 % der Theorie),
R_{f}-Wert: 0.53 (Kieselgel, Methylenchlorid/Methanol/konzentrierte wäßrige Ammoniaklösung = 90:10:0.1)
Massenspektrum (ESI⁻) : m/z = 584, 586 [M-H]⁻

Analog Beispiel II werden folgende Verbindungen erhalten:
(1) 4-[(3-Chlor-4-fluor-phenyl)amino]-6-cyclopentylmethoxy-7-{2-[4-(tert.butyloxycarbonyl)-piperazin-1-yl]-ethoxy}-chinazolin (Die Reaktion wird in Gegenwart von Kaliumcarbonat, Diisopropylethylamin und Benzyl-tributyl-ammoniumchlorid in Dioxan/Wasser (20:1) durchgeführt)
   R_{f}-Wert: 0.55 (Kieselgel, Methylenchlorid/Methanol/konzentrierte wäßrige Ammoniaklösung = 90:10:0.1)
   Massenspektrum (ESI⁻): m/z = 598, 600 [M-H]⁻
(2) 4-[(3-Chlor-4-fluor-phenyl)amino]-6-cyclopropylmethoxy-7-{2-[4-(tert.butyloxycarbonyl)-piperazin-1-yl]-chinazolin
   R_{f}-Wert: 0.43 (Kieselgel, Methylenchlorid/Methanol/konzentrierte wäßrige Ammoniaklösung = 90:10:0.1)
   Massenspektrum (ESI⁻) : m/z = 570, 572 [M-H]⁻
(3) 4-[(3-Chlor-4-fluor-phenyl)amino]-6-[2-(4-{N-[(tert.-butyloxycarbonyl)methyl]-N-(2-hydroxy-ethyl)-amino}-piperidin-1-yl)-ethoxy]-7-methoxy-chinazolin
   (Die Reaktion wird in Gegenwart von Diisopropylethylamin als Hilfsbase durchgeführt.)
   R_{f}-Wert: 0.22 (Kieselgel, Essigester/Methanol/konzentrierte, wäßrige Ammoniaklösung = 90:10:0.5)
   Massenspektrum (ESI⁻): m/z = 602, 604 [M-H]⁻
(4) 4-[(3-Chlor-4-fluor-phenyl)amino]-6-[2-(4-{N-[(tert.-butyloxycarbonyl)methyl]-N-(2-hydroxy-ethyl)-amino}-piperidin-1-yl)-ethoxy]-7-((*R*)-tetrahydrofuran-3-yloxy)-chinazolin
   (Die Reaktion wird in Gegenwart von Diisopropylethylamin als Hilfsbase durchgeführt.)
   R_{f}-Wert: 0.24 (Kieselgel, Essigester/Methanol/konzentrierte, wäßrige Ammoniaklösung = 90:10:0.5)
   Massenspektrum (ESI⁺): m/z = 660, 662 [M+H]⁺

### Beispiel III*

### 4-[(3-Chlor-4-fluor-phenyl)amino]-6-cyclopentylmethoxy-7 (2-brom-ethoxy)-chinazolin

Zu 3.50 g 4-[(3-Chlor-4-fluor-phenyl)amino]-6-cyclopentylmethoxy-7-hydroxy-chinazolin und 6.89 ml 1,2-Dibromethan in 40 ml N,N-Dimethylformamid werden 4.84 g Kaliumcarbonat gegeben. Das Reaktionsgemisch wird unter Stickstoff-Atmosphäre 1.5 Stunden bei 80°C gerührt. Nach Abkühlung auf Raumtemperatur wird das Reaktionsgemisch filtriert und das Filtrat im Vakuum eingeengt. Der ölige, braune Rückstand wird im Eisbad abgekühlt und mit wenig Methanol verrieben, wobei ein gelblicher Feststoff auskristallisiert. Der Niederschlag wird abgesaugt, mit kaltem Methanol nachgewaschen und im Vakuumexsikkator getrocknet.
Ausbeute: 2.60 g (58 % der Theorie),
R_{f}-Wert: 0.82 (Kieselgel, Methylenchlorid/Methanol 9:1) Massenspektrum (ESI⁺): m/z = 494, 496, 498 [M+H]⁺

Analog Beispiel III werden folgende Verbindungen erhalten:
(1) (2) 4-[(3-Chlor-4-fluor-phenyl)amino]-6-cyclopentyloxy-7-(2-brom-ethoxy)-chinazolin
   R_{f}-Wert: 0.65 (Kieselgel, Methylenchlorid/Methanol/konzentrierte wäßrige Ammoniaklösung = 90:10:0.1)
   Massenspektrum (ESI⁻) : m/z = 478, 480, 482 [M-H]⁻
(2) 4-[(3-Chlor-4-fluor-phenyl)amino]-6-cyclopropylmethoxy-7-(2-brom-ethoxy)-chinazolin (Die Reaktion wird in Acetonitril durchgeführt)
   R_{f}-Wert: 0.72 (Kieselgel, Methylenchlorid/Methanol/konzentrierte wäßrige Ammoniaklösung = 90:10:0.1)
   Massenspektrum (ESI⁻): m/z = 464, 466, 468 [M-H]⁻
(3) 4-[(3-Chlor-4-fluor-phenyl)amino]-6-(2-brom-ethoxy)-7-((*R*)-tetrahydrofuran-3-yloxy)-chinazolin
   (Die Reaktion wird in Acetonitril bei 60°C durchgeführt.)
   R_{f}-Wert: 0.37 (Kieselgel, Methylenchlorid/Methanol = 9:1) Massenspektrum (ESI⁻): m/z = 480, 482, 484 [M-H]⁻

### Beispiel IV*

### 4-[(3-Chlor-4-fluor-phenyl)amino]-6-cyclopentylmethoxy-7-hydroxy-chinazolin

4.99 g 4-[(3-Chlor-4-fluor-phenyl)amino]-6-cyclopentylmethoxy-7-methylcarbonyloxy-chinazolin werden in 80 ml Methanol suspendiert und mit 1.80 ml konzentrierter wäßriger Ammoniaklösung versetzt. Das Reaktionsgemisch wird über Nacht bei Raumtemperatur gerührt. Zur Aufarbeitung wird das Reaktionsgemisch mit 500 ml Methylenchlorid verdünnt, mit Wasser und gesättigter Natriumchlorid-Lösung gewaschen, über Magnesiumsulfat getrocknet und eingeengt. Man erhält 4.30 g eines bräunlichen Feststoffes. Das Rohprodukt wird mit tert.Butylmethylether verrührt, abgesaugt, mit wenig tert.Butylmethylether nachgewaschen und bei Raumtemperatur im Vakuum getrocknet.
Ausbeute: 3.59 g (80 % der Theorie),
R_{f}-Wert: 0.48 (Kieselgel, Methylenchlorid/Methanol/konzentrierte wäßrige Ammoniaklösung = 90:10:0.1)
Massenspektrum (ESI⁺) : m/z = 388, 340 [M+H]⁺

Analog Beispiel IV werden folgende Verbindungen erhalten:
(1) 4-[(3-Chlor-4-fluor-phenyl)amino]-6-cyclopentyloxy-7-hydroxy-chinazolin
   R_{f}-Wert: 0.53 (Kieselgel, Methylenchlorid/Methanol/konzentrierte wäßrige Ammoniaklösung = 90:10:0.1)
   Massenspektrum (ESI⁺) : m/z = 374, 376 [M+H]⁺
(2) 4-[(3-Chlor-4-fluor-phenyl)amino]-6-cyclopropylmethoxy-7-hydroxy-chinazolin
   R_{f}-Wert: 0.56 (Kieselgel, Methylenchlorid/Methanol = 9:1) Massenspektrum (ESI⁻) : m/z = 358, 360 [M-H]⁻

### Beispiel V*

### 4-[(3-Chlor-4-fluor-phenyl)amino]-6-cyclopentylmethoxy-7-methylcarbonyloxy-chinazolin

4.03 g 4-Chlor-6-cyclopentylmethoxy-7-methylcarbonyloxy-chinazolin werden in 70 ml Isopropanol suspendiert und mit 1.95 g 3-Chlor-4-fluor-anilin versetzt. Das Reaktionsgemisch wird zwei Stunden unter Stickstoff-Atmosphäre unter Rückfluß erhitzt. Nach Abkühlung auf Raumtemperatur wird der entstandene helle Niederschlag abgesaugt, mit wenig Isopropanol nachgewaschen und an der Luft getrocknet.
Ausbeute: 4.99 g (92 % der Theorie),
R_{f}-Wert: 0.80 (Kieselgel, Methylenchlorid/Methanol/konzentrierte wäßrige Ammoniaklösung = 90:10:0.1)
Massenspektrum (ESI⁺) : m/z = 430, 432 [M+H]⁺

Analog Beispiel V werden folgende Verbindungen erhalten:
(1) 4-[(3-Chlor-4-fluor-phenyl)amino]-6-cyclopentyloxy-7-methylcarbonyloxy-chinazolin
   R_{f}-Wert: 0.73 (Kieselgel, Methylenchlorid/Methanol/konzentrierte wäßrige Ammoniaklösung = 90:10:0.1)
   Massenspektrum (ESI⁺) : m/z = 416, 418 [M+H]⁺
(2) 4-[(3-Chlor-4-fluor-phenyl)amino]-6-cyclopropylmethoxy-7-methylcarbonyloxy-chinazolin
   R_{f}-Wert: 0.86 (Kieselgel, Methylenchlorid/Methanol = 9:1) Massenspektrum (ESI⁺): m/z = 402, 404 [M+H]⁺

### Beispiel VI*

### 4-Chlor-6-cyclopentylmethoxy-7-methylcarbonyloxy-chinazolin

3.80 g 4-Hydroxy-6-cyclopentylmethoxy-7-methylcarbonyloxy-chinazolin werden in 90 ml Thionylchlorid suspendiert und unter Stickstoff-Atmosphäre zum Sieden erhitzt. Nach Zugabe von vier Tropfen N,N-Dimethylformamid wird das Reaktionsgemisch noch zwei Stunden unter Rückfluß erhitzt. Nach Abkühlung auf Raumtemperatur wird das überschüssige Thionylchlorid im Wasserstrahlvakuum abdestilliert. Der braune Rückstand wird mit 30 ml Toluol verrührt. Das Lösungsmittel wird abdestilliert und es bleiben 4.30 g eines graubraunen Feststoffe zurück, welcher ohne weitere Reinigung weiter umgesetzt wird. R_{f}-Wert: 0.89 (Kieselgel, Methylenchlorid/Methanol/konzentrierte wäßrige Ammoniaklösung = 90:10:0.1)

Analog Beispiel VI werden folgende Verbindungen erhalten:
(1) 4-Chlor-6-cyclopentyloxy-7-methylcarbonyloxy-chinazolin
   R_{f}-Wert: 0.69 (Kieselgel, Methylenchlorid/Methanol/konzentrierte wäßrige Ammoniaklösung = 90:10:0.1)
(2) 4-Chlor-6-cyclopropylmethoxy-7-methylcarbonyloxy-chinazolin
   R_{f}-Wert: 0.84 (Kieselgel, Methylenchlorid/Methanol = 9:1)

### Beispiel VII*

### 4-Hydroxy-6-cyclopentylmethoxy-7-methylcarbonyloxy-chinazolin

4.30 g 4,7-Dihydroxy-6-cyclopentylmethoxy-chinazolin in 100 ml Pyridin werden unter Stickstoff-Atmosphäre auf 80°C erhitzt. Zur dunkelbraunen Suspension werden 1.80 ml Essigsäureanhydrid gegeben. Das Reaktionsgemisch wird drei Stunden bei 80°C gerührt, wobei eine vollständige Lösung entsteht. Nach Abkühlung auf Raumtemperatur wird das Reaktionsgemisch auf ca. 800 ml Eiswasser gegossen. Der entstandene Niederschlag wird abgesaugt und gründlich mit Wasser nachgewaschen. Der hellgraue Feststoff wird im Vakuumexsikkator getrocknet.
Ausbeute: 3.82 g (77% der Theorie),
R_{f}-Wert: 0.49 (Kieselgel, Methylenchlorid/Methanol = 9:1) Massenspektrum (ESI⁻) : m/z = 301 [M-H]⁻

Analog Beispiel VII werden folgende Verbindungen erhalten:
(1) 4-Hydroxy-6-cyclopentyloxy-7-methylcarbonyloxy-chinazolin Schmelzpunkt: 209-212°C
   Massenspektrum (ESI⁻) : m/z = 287 [M-H]⁻
(2) 4-Hydroxy-6-cyclopropylmethoxy-7-methylcarbonyloxy-chinazolin
   R_{f}-Wert: 0.53 (Kieselgel, Methylenchlorid/Methanol = 9:1)
   Massenspektrum (ESI⁻) : m/z = 273 [M-H]⁻

### Beispiel VIII*

### 4,7-Dihydroxy-6-cyclopentylmethoxy-chinazoline

5.76 g 2-Amino-5-cyclopentylmethoxy-4-hydroxy-benzoesäure und 6.52 g Formamidinacetat in 140 ml Ethanol werden ca. drei Stunden unter Rückfluß erhitzt. Zur Aufarbeitung wird das Reaktionsgemisch auf etwa 100 ml eingeengt und mit 300 ml Eiswasser versetzt, wobei ein grauer Niederschlag ausfällt. Der Niederschlag wird abgesaugt, mit Wasser nachgewaschen und im Vakuumexsikkator getrocknet.
Ausbeute: 4.57 g (77 % der Theorie),
R_{f}-Wert: 0.25 (Kieselgel, Methylenchlorid/Methanol = 95:5) Massenspektrum (ESI⁻) : m/z = 259 [M-H]⁻

Analog Beispiel VIII werden folgende Verbindungen erhalten:
(1) 4,7-Dihydroxy-6-cyclopentyloxy-chinazolin
   R_{f}-Wert: 0.42 (Kieselgel, Methylenchlorid/Methanol/konzentrierte wäßrige Ammoniaklösung = 90:10:0.1)
   Massenspektrum (EI) : m/z = 246 [M]⁺
(2) 4,7-Dihydroxy-6-cyclopropylmethoxy-chinazolin
   R_{f}-Wert: 0.45 (Kieselgel, Methylenchlorid/Methanol/konzentrierte wäßrige Ammoniaklösung = 90:10:0.1)
   Massenspektrum (ESI⁻): m/z = 231 [M-H]⁻

### Beispiel IX*

### 2-Amino-5-cyclopentylmethoxy-4-hydroxy-benzoesäure

6.50 g 5-Cyclopentylmethoxy-4-hydroxy-2-nitro-benzoesäure werden in 130 ml Methanol gelöst, mit 2.00 g Raney-Nickel versetzt und unter einem Wasserstoffdruck von 50 psi etwa drei Stunden bei Raumtemperatur hydriert, bis die berechnete Menge Wasserstoff aufgenommen ist. Der Katalysator wird abfiltriert und mit heißem Methanol nachgewaschen. Das Filtrat wird im Vakuum eingeengt. Es bleibt ein bräunlicher Feststoff zurück, welcher ohne weitere Reinigung weiter umgesetzt wird.
Ausbeute: 5.79 g (100 % der Theorie),
R_{f}-Wert: 0.67 (Kieselgel, Methylenchlorid/Methanol = 9:1) Massenspektrum (ESI⁻): m/z = 250 [M-H]⁻

Analog Beispiel IX werden folgende Verbindungen erhalten:
(1) 2-Amino-5-cyclopentyloxy-4-hydroxy-benzoesäure
   R_{f}-Wert: 0.38 (Kieselgel, Methylenchlorid/Methanol/konzentrierte wäßrige Ammoniaklösung = 90:10:0.1)
   Massenspektrum (ESI⁺) : m/z = 238 [M+H]⁺
(2) 2-Amino-5-cyclopropylmethoxy-4-hydroxy-benzoesäure
   R_{f}-Wert: 0.51 (Kieselgel, Methylenchlorid/Methanol/konzentrierte wäßrige Ammoniaklösung = 90:10:0.1)
   Massenspektrum (ESI⁻): m/z = 222 [M-H]⁻

### Beispiel X*

### 5-cyclopentylmethoxy-4-hydroxy-2-nitro-benzoesäure

15.37 g 4,5-Methylendioxy-2-nitro-benzoesäure und 51.84 ml Cyclopentylmethanol werden in 100 ml Dimethylsulfoxid gelöst und unter Stickstoff-Atmosphäre im Eisbad abgekühlt. Nun werden portionsweise 3.90 g Natrium zugegeben. Das Reaktionsgemisch wird 30 Minuten unter Eisbad-Kühlung gerührt, dann kurzzeitig auf 35-40°C erwärmt und anschließend noch weitere drei Stunden unter Eisbad-Kühlung gerührt. Anschließend wird das Eisbad entfernt und das Reaktionsgemisch über Nacht bei Raumtemperatur gerührt. Die dunkelbraunrote Reaktionslösung wird auf ca. 800 ml Aceton gegossen, wobei ein dunkelbrauner Niederschlag ausfällt. Der Niederschlag wird abgesaugt, mit Aceton nachgewaschen, in 300-400 ml Wasser gelöst und mit 60 ml 2N Salzsäure auf etwa pH 2 eingestellt. Die wäßrige Lösung wird mehrmals mit Methylenchlorid extrahiert. Die vereinigten Extrakte werden mit gesättigter Natriumchlorid-Lösung gewaschen, über Natriumsulfat getrocknet und eingeengt. Der dunkelbraune, ölige Kolbenrückstand wird in 800 ml Methylenchlorid gelöst und über ein Kieselgelpackung mit Methylenchlorid/Methanol (9:1) gereinigt. Man erhält ein braunes Öl, welches durch verrühren mit Wasser unter Eisbad-Kühlung zur Kristallisation gebracht wird. Der entstandene bräunliche Niederschlag wird abgesaugt, mit wenig Wasser nachgewaschen und im Vakuumexsikkator getrocknet.
Ausbeute: 9.55 g (47 % der Theorie),
R_{f}-Wert: 0.67 (Kieselgel, Toluol/Dioxan/Ethanol/Eisessig = 90:10:10:6)
Massenspektrum (ESI⁻): m/z = 280 [M-H]⁻

Analog Beispiel X werden folgende Verbindungen erhalten:
(1) 5-Cyclopentyloxy-4-hydroxy-2-nitro-benzoesäure
   R_{f}-Wert: 0.62 (Kieselgel, Toluol/Dioxan/Ethanol/Eisessig = 90:10:10:6)
   Massenspektrum (ESI⁻): m/z = 266 [M-H]⁻
(2) 5-Cyclopropylmethoxy-4-hydroxy-2-nitro-benzoesäure
   R_{f}-Wert: 0.61 (Kieselgel, Toluol/Dioxan/Ethanol/Eisessig = 90:10:10:6)
   Massenspektrum (ESI⁻): m/z = 252 [M-H]⁻

### Beispiel XI*

### 8-(tert.Butyloxycarbonyl)-perhydro-pyrazino[2,1-c][1,4]oxazin-3-on

2.00 g 1-(tert.Butyloxycarbonyl)-4-[(ethoxycarbonyl)methyl]-3-hydroxymethyl-piperazin in 2.5 ml Acetonitril werden mit 500 mg p-Toluolsulfonsäure-monohydrat versetzt. Das Reaktionsgemisch wird drei Stunden unter Rückfluß erhitzt, bis die Umsetzung vollständig ist. Anschließend wird das Lösungsmittel im Vakuum abdestilliert. Das Rohprodukt wird ohne weitere Reinigung direkt weiter umgesetzt.
R_{f}-Wert: 0.80 (Kieselgel, Essigester/Methanol = 9:1)

### Beispiel XII*

### 1-(tert.Butyloxycarbonyl)-4-[(ethoxycarbonyl)methyl]-3-hydroxymethyl-piperazin und 8-(tert.Butyloxycarbonyl)-perhydropyrazino[2,1-c][1,4]oxazine-3-on

Zu 5.80 g 1-(tert.Butyloxycarbonyl)-3-hydroxymethyl-piperazin und 4.50 ml Triethylamin in 60 ml Acetonitril werden 3.90 ml Bromessigsäureethylester gegeben. Das Reaktionsgemisch wird über Nacht unter Rückfluß erhitzt, wobei laut Dünnschichtchromatographie zwei Produkte entstehen. Zur Aufarbeitung wird das Reaktionsgemisch im Vakuum eingeengt und der Rückstand zwischen Essigester und Wasser verteilt. Die organische Phase wird über Magnesiumsulfat getrocknet, eingeengt und über eine Kieselgelsäule mit Essigester/Methanol (97:3) chromatographiert. Man erhält die beiden folgenden Produkte als gelbliche Öle:
8-(tert.Butyloxycarbonyl)-perhydro-pyrazino[2,1-c][1,4]oxazin-3-on
Ausbeute: 3.43 g (50 % der Theorie),
R_{f}-Wert: 0.80 (Kieselgel, Essigester/Methanol = 9:1) 1-(tert.Butyloxycarbonyl)-4-[(ethoxycarbonyl)methyl]-3-hydroxymethyl-piperazin
Aubeute: 2.08 g (26 % der Theorie),
R_{f}-Wert: 0.58 (Kieselgel, Essigester/Methanol = 9:1)
Massenspektrum (ESI⁺) : m/z = 303 [M+H]⁺

### Beispiel XIII*

### 1-(tert.Butyloxycarbonyl)-3-hydroxymethyl-piperazin

Eine Suspension aus 900 mg Lithiumborhydrid in 20 ml Tetrahydrofuran wird tropfenweise mit einer Lösung aus 8.00 g 1-(tert.Butyloxycarbonyl)-3-ethoxycarbonyl-piperazin in 10 ml Tetrahydrofuran versetzt und anschließend drei Stunden unter Rückfluß erhitzt. Zur Aufarbeitung wird das Reaktionsgemisch eingeengt, mit 10%iger wäßriger Zitronensäurelösung auf pH 4 eingestellt und etwa 40 Minuten unter Eisbad-Kühlung gerührt. Anschließend wird das Gemisch mit konzentrierter Natronlauge alkalisch gestellt und über Nacht stehengelassen. Am nächsten Morgen wird es mit tert.Butylmethylether extrahiert. Die organische Phase wird über Magnesiumsulfat getrocknet und eingeengt. Es bleibt ein klares Öl zurück, welches langsam kristallisiert.
Ausbeute: 5.80 g (87 % der Theorie),
R_{f}-Wert: 0.28 (Kieselgel, Essigester/Methanol = 4:1) Massenspektrum (ESI⁺) : m/z = 217 [M+H]⁺

### Beispiel XIV*

### 1-(tert.Butyloxycarbonyl)-3-ethoxycarbonyl-piperazin

Zu 15.80 g 2-Ethoxycarbonyl-piperazin in 400 ml Ethanol werden unter Eisbad-Kühlung 21.80 g Pyrokohlensäure-di-tert.butylester gegeben. Das Reaktionsgemisch wird noch drei Stunden bei 0°C gerührt. Anschließend wird es eingeengt und der Rückstand zwischen Essigester und Wasser verteilt. Die organische Phase wird über Magnesiumsulfat getrocknet, eingeengt und chromatographisch über eine Kieselgelsäule mit Essigester/Methanol (95:5) als Laufmittel gereinigt.
Ausbeute: 24.30 g (94 % der Theorie),
R_{f}-Wert: 0.40 (Kieselgel, Essigester/Methanol = 9:1) Massenspektrum (ESI⁺): m/z = 281 [M+Na]⁺

### Beispiel XV*

### 4-{N-[(tert.-Butyloxycarbonyl)methyl]-N-(2-hydroxy-ethyl)-amino}-piperidin

Die Verbindung wird durch Hydrierung von 1-Benzyloxycarbonyl-4-{N-[(tert.-butyloxycarbonyl)methyl]-N-(2-hydroxy-ethyl)-amino}-piperidin in Ethanol in Gegenwart von 10% Palladium auf Aktivkohle in einer Parr-Apparatur erhalten.
Massenspektrum (ESI⁺): m/z = 259 [M+H]⁺

### Beispiel XVI*

### 1-Benzyloxycarbonyl-4-{N-[(tert.-butyloxycarbonyl)methyl]-N-(2-hydroxy-ethyl)-amino}-piperidin

4.89 g 1-Benzyloxycarbonyl-4-oxo-piperidin und 3.67 g (2-Hydroxy-ethylamino)-essigsäure-tert.-butylester in 100 ml Methylenchlorid werden mit 1.2 ml Eisessig versetzt und in einem Eiswasserbad abgekühlt. Nun werden insgesamt 4.44 g Natriumtriacetoxyborhydrid portionsweise über einen Zeitraum von einer Stunde zugegeben. Man läßt das Reaktionsgemisch über Nacht auf Raumtemperatur erwärmen. Zur Aufarbeitung wird das Gemisch mit gesättigter Natriumhydrogencarbonat-Lösung versetzt. Die organische Phase wird abgetrennt, über Magnesiumsulfat getrocknet und eingeengt. Das Rohprodukt wird chromatographisch über eine Kieselgelsäule mit Essigester/Petrolether (1:1) als Laufmittel gereinigt.
Ausbeute: 3.52 g (43 % der Theorie)
R_{f}-Wert: 0.40 (Kieselgel, Cyclohexan/Essigester = 1:1) Massenspektrum (ESI⁺): m/z = 393 [M+H]⁺

### Beispiel XVII*

### 4-[(3-Chlor-4-fluor-phenyl)amino]-6-hydroxy-7-((R)-tetrahydrofuran-3-yloxy)-chinazolin

Die Verbindung wird durch Behandeln von 4-[(3-Chlor-4-fluor-phenyl)amino]-6-benzyloxy-7-((*R*)-tetrahydrofuran-3-yloxy)-chinazolin mit Trifluoressigsäure unter Rückfluß erhalten. R_{f}-Wert: 0.32 (Kieselgel, Methylenchlorid/Methanol = 9:1)

### Beispiel XVIII*

### 4-[(3-Chlor-4-fluor-phenyl)amino]-6-benzyloxy-7-((R)-tetrahydrofuran-3-yloxy) chinazolin

Zu einer Lösung aus 8.00 g 4-[(3-Chlor-4-fluor-phenyl)amino]-6-benzyloxy-7-hydroxy-chinazolin (siehe WO 0055141 A1) und 2.42 ml (S)-(+)-3-Hydroxy-tetrahydrofuran und 7.95 g Triphenylphosphin in 160 ml Tetrahydrofuran werden 5.03 ml Azodicarbonsäurediethylester getropft. Das Reaktionsgemisch wird über Nacht bei Raumtemperatur gerührt und anschließend am Rotationsverdampfer eingeengt. Der Kolbenrückstand wird chromatographisch über eine Kieselgelsäule mit Methylenchlorid/Essigester (Gradient von 2:1 auf 1:2) als Laufmittel gereinigt.
Ausbeute: 7.34 g (78 % der Theorie)
Schmelzpunkt: 165-168°C
Massenspektrum (ESI⁺): m/z = 466, 468 [M+H]⁺

### Beispiel XIX*

### 2-Oxo-3-{[1-(tert.-butyloxycarbonyl)-piperidin-4-yl]sulfanyl}-tetrahydrofuran

Die Verbindung wird durch Umsetzung von 1-(tert.-Butyloxycarbonyl)-4-mercapto-piperidin mit 3-Brom-dihydro-furan-2-on in N,N-Dimethylformamid in Gegenwart von Kalium-tert.-butylat erhalten.
R_{f}-Wert: 0.35 (Kieselgel, Cyclohexan/Essigester = 3:2)
Massenspektrum (ESI⁻) : m/z = 300 [M-H]⁻

Herstellung der Endverbindungen:
(Alle in der Folge mit* bezeichneten Beispiele sind nicht Teil der Erfindung)

### Beispiel 1*

### 4-[(3-Chlor-4-fluor-phenyl)amino]-6-cyclopentyloxy-7-{2-[4-(2-oxo-tetrahydrofuran-3-yl)-piperazin-1-yl]-ethoxy}-chinazolin

Zu 180 mg 4-[(3-Chlor-4-fluor-phenyl)amino]-6-cyclopentyloxy-7-[2-(piperazin-1-yl)-ethoxy]-chinazolin und 0.14 ml Triethylamin in 4 ml Tetrahydrofuran werden 67 mg 3-Brom-dihydrofuran-2-on gegeben. Das Reaktionsgemisch wird übers Wochenende bei Raumtemperatur gerührt. Zur Aufarbeitung wird das Reaktionsgemisch am Rotationsverdampfer im Vakuum eingeengt. Der Rückstand wird über eine Kieselgelsäule mit Methylenchlorid/Methanol (95:5 bis 90:10) chromatographiert. Der so erhaltene helle Feststoff wird mit Diethylether verrührt, abgesaugt und in einer Trockenpistole bei 60°C im Vakuum getrocknet.
Ausbeute: 120 mg ( % der Theorie),
R_{f}-Wert: 0.38 (Kieselgel, Methylenchlorid/Methanol/konzentrierte wäßrige Ammoniaklösung = 90:10:0.1)
Massenspektrum (ESI⁻) : m/z = 568, 570 [M-H]⁻

### Beispiel 2*

### 4-[(3-Chlor-4-fluor-phenyl)amino]-6-cyclopentylmethoxy-7-(2-{4-[(S)-(2-oxo-tetrahydrofuran-5-yl)carbonyl]-piperazin-1-yl}-ethoxy)-chinazolin

72 mg (*S*)-(+)-5-Oxo-tetrahydrofuran-2-carbonsäure werden in 2.5 ml N,N-Dimethylformamid gelöst, mit 183 mg (Benzotriazol-1-yl) -N,N,N',N'-tetramethyl-uronium-tetrafluoroborat versetzt und 30 Minuten bei Raumtemperatur gerührt. Diese Lösung wird anschließend zu einer Mischung aus 250 mg 4-[(3-Chlor-4-fluor-phenyl) amino] -6-cyclopentylmethoxy-7- [2- (piperazin-1-yl) - ethoxy]-chinazolin und 110 µl Triethylamin in 2.5 ml N,N-Dimethylformamid gegeben. Das Reaktionsgemisch wird fünf Stunden bei Raumtemperatur gerührt. Zur Aufarbeitung wird das Gemisch auf 50 ml Wasser gegossen. Es fällt ein weißer Niederschlag aus, welcher abgesaugt und mit Wasser nachgewaschen wird. Das Rohprodukt wird chromatographisch über eine Alox-Säule (Aktivitätsstufe III) mit Methylenchlorid/Methanol (98:2) als Laufmittel gereinigt. Man erhält das gewünschte Produkt als hellen Feststoff.
Ausbeute: 78 mg (26 % der Theorie),
R_{f}-Wert: 0.46 (Kieselgel, Methylenchlorid/Methanol/konzentrierte wäßrige Ammoniaklösung = 90:10:0.1)
Massenspektrum (ESI⁻): m/z = 610, 612 [M-H]⁻

Analog Beispiel 2 wird folgende Verbindung erhalten:
(1) 4-[(3-Chlor-4-fluor-phenyl)aminol-6-cyclopentyloxy-7-(2-{4-[(*S*)-(2-oxo-tetrahydrofuran-5-yl)carbonyl]-piperazin-1-yl}-ethoxy)-chinazolin
   R_{f}-Wert: 0.37 (Kieselgel, Methylenchlorid/Methanol/konzentrierte wäßrige Ammoniaklösung = 90:10:0.1)
   Massenspektrum (ESI⁻): m/z = 596, 598 [M-H]⁻

### Beispiel 3* (Alle in der Folge mit* bezeichneten Beispiele sind nicht Teil der Erfindung)

### 4-[(3-Chlor-4-fluor-phenyl)amino]-6-cyclopentylmethoxy-7-{2-[4-(2-oxo-tetrahydrofuran-4-yl)-piperazin-1-yl]-ethoxy}-chinazolin

Zu einer Lösung aus 230 mg 4-[(3-Chlor-4-fluor-phenyl)amino]-6-cyclopentylmethoxy-7-[2-(piperazin-1-yl)-ethoxy]-chinazolin in 2 ml Methanol werden 46 mg (5H)-Furan-2-on gegeben. Das Reaktionsgemisch wird 24 Stunden bei Raumtemperatur, dann weitere sechs Stunden bei 50°C gerührt. Es werden insgesamt nochmals sechs Tropfen (5H)-Furan-2-on zugegeben, bis die Umsetzung vollständig ist. Das Lösungsmittel wird am Rotationsverdampfer abdestilliert und das Rohprodukt chromatograpisch über eine Alox-Säule (Aktivitätsstufe III) mit Methylenchlorid/Methanol (98:2) als Laufmittel gereinigt. Man erhält das gewünschte Produkt als farblosen Feststoff.
Ausbeuten 106 mg (40 % der Theorie),
R_{f}-Wert: 0.50 (Kieselgel, Methylenchlorid/Methanol/konzentrierte wäßrige Ammoniaklösung = 90:10:0.1)
Massenspektrum (ESI⁻) : m/z = 582, 584 [M-H]⁻

Analog Beispiel 3 werden folgende Verbindungen erhalten:
(1) 4-[(3-Chlor-4-fluor-phenyl)amino]-6-cyclopentyloxy-7-{2-[4-(2-oxo-tetrahydrofuran-4-yl)-piperazin-1-yl]-ethoxy}-chinazolin
   R_{f}-Wert: 0.42 (Kieselgel, Methylenchlorid/Methanol/konzentrierte wäßrige Ammoniaklösung = 90:10:1)
   Massenspektrum (ESI⁻) : m/z = 468, 470 [M-H]⁻
(2) 4-[(3-Chlor-4-fluor-phenyl)amino]-6-cyclopropylmethoxy-7-{2-[4-(2-oxo-tetrahydrofuran-4-yl)-piperazin-1-yl]-ethoxy}-chinazolin
   R_{f}-Wert: 0.35 (Kieselgel, Methylenchlorid/Methanol/konzentrierte wäßrige Ammoniaklösung = 90:10:1)
   Massenspektrum (ESI⁻) : m/z = 554, 556 [M-H]⁻

### Beispiel 4* (Alle in der Folge mit* bezeichneten Beispiele sind nicht Teil der Erfindung)

### 4-[(3-Chlor-4-fluor-phenyl)amino]-6-cyclopentyloxy-7-(2-{4-[(R)-(2-oxo-tetrahydrofuran-5-yl)methyl]-piperazin-1-yl}-ethoxy)-chinazolin

Zu 300 mg 4-[(3-Chlor-4-fluor-phenyl)amino]-6-cyclopentyloxy-7-[2-(piperazin-1-yl)-ethoxy]-chinazolin in 20 ml Acetonitril werden 160 mg Kaliumcarbonat und 50 mg Natriumiodid gegeben. Dann werden 170 mg (R)-5-[(Methansulfonyloxy)methyl]-2-oxo-tetrahydrofuran zugegeben. Das Reaktionsgemisch wird vier Stunden unter Rückfluß erhitzt, dann werden nochmals 0.10 g (R)-5-[(Methansulfonyloxy)methyl]-2-oxo-tetrahydrofuran zugesetzt. Nach weiteren zehn Stunden unter Rückfluß werden erneut 0.12 g (*R*)-5-[(Methansulfonyloxy)methyl]-2-oxo-tetrahydrofuran sowie 0.20 g Kaliumcarbonat und 70 mg Natriumiodid zugegeben. Das Reaktionsgemisch wird noch fünf Stunden unter Rückfluß gekocht und anschließend übers Wochenende stehengelassen. Zur Aufarbeitung wird das Reaktionsgemisch filtriert und das Filtrat eingeengt. Das Rohprodukt wird chromatographisch über eine Kieselgelsäule mit Methylenchlorid/Methanol/konzentrierter wäßriger Ammoniaklösung (95:5:0.05, später 93:7:0.1) als Laufmittel gereinigt. Man erhält die Titelverbindung als weißen Feststoff.
Ausbeute: 170 mg (47 % der Theorie),
R_{f}-Wert: 0.35 (Kieselgel, Methylenchlorid/Methanol/konzentrierte wäßrige Ammoniaklösung = 90:10:1)
Massenspektrum (ESI⁻) : m/z = 582, 584 [M-H]⁻

Analog Beispiel 4 werden folgende Verbindungen erhalten:
(1) 4-[(3-Chlor-4-fluor-phenyl)amino]-6-cyclopentylmethoxy-7-(2-(4-[(R)-(2-oxo-tetrahydrofuran-5-yl)methyl]-piperazin-1-yl}-ethoxy)-chinazolin
   R_{f}-Wert: 0.50 (Kieselgel, Methylenchlorid/Methanol/konzentrierte wäßrige Ammoniaklösung = 90:10:0.1)
   Massenspektrum (ESI⁻) : m/z = 596, 598 [M-H]⁻
(2) 4-[(3-Chlor-4-fluor-phenyl)amino]-6-cyclopropylmethoxy-7-(2-{4-[(*R*)-(2-oxo-tetrahydrofuran-5-yl)methyl]-piperazin-1-yl}-ethoxy)-chinazolin
   R_{f}-Wert: 0.36 (Kieselgel, Methylenchlorid/Methanol/konzentrierte wäßrige Ammoniaklösung = 90:10:1)
   Massenspektrum (ESI⁻): m/z = 568, 570 [M-H]⁻

### Beispiel 5* (Alle in der Folge mit* bezeichneten Beispiele sind nicht Teil der Erfindung)

### 4-[(3-Chlor-4-fluor-phenyl)amino]-6-cyclopropylmethoxy-7-[2-(3-oxo-perhydro-pyrazino[2,1-c][1,4]oxazin-8-yl)-ethoxy]-chinazolin

Zu 150 mg 4-[(3-Chlor-4-fluor-phenyl)amino]-6-cyclopropylmethoxy-7-(2-brom-ethoxy)-chinazolin in 15 ml Acetonitril werden 0.25 ml Diisopropylethylamin und 260 mg Perhydro-pyrazino[2,1-c][1,4]oxazin-3-on x 2 Trifluoressigsäure gegeben. Das Reaktionsgemisch wird eine Stunde bei Raumtemperatur gerührt und anschließend zwei Stunden unter Rückfluß erhitzt. Dann werden 70 mg Kaliumcarbonat und 75 mg Natriumiodid zugegeben. Das Reaktionsgemisch wird noch etwa 14 Stunden unter Rückfluß erhitzt, dabei werden sukzessive insgesamt weitere 175 mg Perhydro-pyrazino[2,1-*c*][1,4]oxazin-3-on x 2 Trifluoressigsäure und 300 mg Kaliumcarbonat zugesetzt, bis die Umsetzung vollständig ist. Zur Aufarbeitung werden die anorganischen Salze abfiltriert und das Filtrat wird im Vakuum eingeengt. Der Kolbenrückstand wird über eine Kieselgelsäule mit Methylenchlorid/Methanol (95:5) als Laufmittel chromatogräphiert. Man erhält das gewünschte Produkt als hellbraunes Harz.
Ausbeute: 27 mg (16 % der Theorie),
R_{f}-Wert: 0.50 (Kieselgel, Methylenchlorid/Methanol/konzentrierte wäßrige Ammoniaklösung = 90:10:0.1)
Massenspektrum (EI): m/z = 541, 543 [M]⁺

Analog Beispiel 5 wird folgende Verbindung erhalten:
(1) 4-[(3-Chlor-4-fluor-phenyl)amino]-6-(2-{4-[(2-oxo-tetrahydrofuran-3-yl)sulfanyl]-piperidin-1-yl}-ethoxy)-7-methoxy-chinazolin
   R_{f}-Wert: 0.42 (Kieselgel, Essigester/Methanol/konzentrierte, wäßrige Ammoniaklösung = 90:10:0.5)
   Massenspektrum (EI): m/z = 546, 548 [M]⁺

### Beispiel 6

### 4-[(3-Chlor-4-fluor-phenyl)amino]-6-{2-[4-(2-oxo-morpholin-4-yl)-piperidin-1-yl]-ethoxy}-7-methoxy-chinazolin

Die Verbindung wird durch Behandeln von 4-[(3-Chlor-4-fluor-phenyl)amino]-6-[2-(4-{N-[(tert.-butyloxycarbonyl)methyl]-N-(2-hydroxy-ethyl)-amino}-piperidin-1-yl)-ethoxy]-7-methoxy-chinazolin mit Trifluoressigsäure in Acetonitril unter Rückfluß erhalten.
R_{f}-Wert: 0.10 (Kieselgel, Essigester/Methanol/konzentrierte, wäßrige Ammoniaklösung = 90:10:0.5)
Massenspektrum (ESI⁻): m/z = 528, 530 [M-H]⁻

Analog Beispiel 6 wird folgende Verbindung erhalten:
(1) 4-[(3-Chlor-4-fluor-phenyl)amino]-6-{2-[4-(2-oxo-morpholin-4-yl)-piperidin-1-yl]-ethoxy}-7-((*R*)-tetrahydrofuran-3-yloxy)-chinazolin
   R_{f}-Wert: 0.11 (Kieselgel, Essigester/Methanol/konzentrierte, wäßrige Ammoniaklösung = 90:10:0.5)
   Massenspektrum (ESI⁻): m/z = 584, 586 [M-H]⁻

Analog den vorstehenden Beispielen werden folgende Verbindungen erhalten:
(Alle in der Folge mit* bezeichneten Verbindungen sind nicht Teil der Erfindung)
(1)* 4-[(3-Chlor-4-fluor-phenyl)amino)-7-methoxy-6-{3-[4-(2-oxo-tetrahydrofuran-4-yl)-piperazin-1-yl]-propyloxy}-chinazolin
(2)* 4-[(3-Chlor-4-fluor-phenyl)amino]-7-methoxy-6-(3-{4-[(2-oxo-tetrahydrofuran-5-yl)methyl]-piperazin-1-yl}-propyloxy)-chinazolin
(3)* 4-[(3-Chlor-4-fluor-phenyl)amino]-7-methoxy-6-(3-{4-[(2-oxo-tetrahydrofuran-5-yl)carbonyl]-piperazin-1-yl}-propyloxy)-chinazolin
(4)* 4-[(3-Chlor-4-fluor-phenyl)amino]-7-methoxy-6-(3-{4-{2-[(2-oxo-tetrahydrofuran-3-yl)sulfanyl]-ethyl}-piperazin-1-yl}-propyloxy)-chinazolin
(5)* 4-[(3-Chlor-4-fluor-phenyl)amino]-7-methoxy-6-{3-[1-(2-oxo-tetrahydrofuran-4-yl)-piperidin-4-yl]-propyloxy}-chinazolin
(6)* 4-[(3-Chlor-4-fluor-phenyl)amino]-7-methoxy-6-[3-(3-oxo-perhydro-pyrazino[2,1-c][1,4]oxazin-8-yl)-propyloxy]-chinazolin
(7)* 4-[(3-Chlor-4-fluor-phenyl)amino]-7-methoxy-6-[3-(1-oxo-perhydro-pyrazino[2,1-c][1,4]oxazin-8-yl)-propyloxy]-chinazolin
(8)* 4-[(3-Chlor-4-fluor-phenyl)amino]-7-methoxy-6-[3-(2-oxa-3-oxo-8-aza-spiro[4.5]dec-8-yl)-propyloxy]-chinazolin
(9)* 4-[(3-Chlor-4-fluor-phenyl)amino]-7-methoxy-6-[3-(3-oxa-2-oxo-9-aza-spiro[5.5]undecan-9-yl)-propyloxy]-chinazolin
(10)* 4-[(3-Chlor-4-fluor-phenyl)amino]-7-cyclopropylmethoxy-6-[3-(3-oxa-2-oxo-9-aza-spiro[5.5]undecan-9-yl)-propyloxy]-chinazolin
(11)* 4-[(3-Chlor-4-fluor-phenyl)amino]-7-methoxy-6-[2-(3-oxa-2-oxo-9-aza-spiro[5.5]undecan-9-yl)-ethoxy]-chinazolin
(12)* 4-[(R)-(1-Phenyl-ethyl)amino]-7-methoxy-6-[2-(3-oxa-2-oxo-9-aza-spiro[5.5]undecan-9-yl)-ethoxy]-chinazolin
(13)* 4-[(3-Chlor-4-fluor-phenyl)amino]-7-methoxy-6-[3-(1,4-di-oxa-2-oxo-9-aza-spiro[5.5]undecan-9-yl)-propyloxy]-chinazolin
(14)* 4-[(3-Chlor-4-fluor-phenyl)amino]-7-methoxy-6-[3-(4-methyl-1-oxa-2-oxo-4,9-diaza-spiro[5.5]undecan-9-yl)-propyloxy]-chinazolin
(15)* 4-[(3-Chlor-4-fluor-phenyl)amino]-7-cyclopropylmethoxy-6-[3-(4-methyl-1-oxa-2-oxo-4,9-diaza-spiro[5.5]undecan-9-yl)-propyloxy]-chinazolin
(16)* 4-[(3-Chlor-4-fluor-phenyl)amino]-7-cyclopropylmethoxy-6-[2-(4-methyl-1-oxa-2-oxo-4,9-diaza-spiro[5.5]undecan-9-yl)-ethoxy]-chinazolin
(17) 4-[(3-Chlor-4-fluor-phenyl)amino]-7-methoxy-6-{3-[4-(2-oxo-morpholin-4-yl)-piperidin-1-yl]-propyloxy}-chinazolin
(18)* 4-[(3-Chlor-4-fluor-phenyl)amino]-7-methoxy-6-{2-[4-(2-oxo-morpholin-4-yl)-piperidin-1-yl]-ethoxy}-chinazolin
(19) 4-[(3-Chlor-4-fluor-phenyl)amino]-7-methoxy-6-{3-[4-(6-methyl-2-oxo-morpholin-4-yl)-piperidin-1-yl]-propyloxy}-chinazolin
(20) 4-[(3-Chlor-4-fluor-phenyl)amino]-7-cyclopropylmethoxy-6-{3-[4-(6-methyl-2-oxo-morpholin-4-yl)-piperidin-1-yl]-propyloxy}-chinazolin
(21) 4-[(3-Chlor-4-fluor-phenyl)amino]-7-methoxy-6-(3-{4-[(6-methyl-2-oxo-morpholin-4-yl)methyl]-piperidin-1-yl}-propyloxy)-chinazolin
(22)* 4-[(3-Chlor-4-fluor-phenyl)amino]-7-methoxy-6-(3-{4-[(2-oxo-tetrahydrofuran-3-yl)sulfanyl]-piperidin-1-yl}-propyloxy)-chinazolin
(23)* 4-[(3-Chlor-4-fluor-phenyl)amino]-7-methoxy-6-[3-(6-methoxymethyl-2-oxo-morpholin-4-yl)-propyloxy]-chinazolin
(24)* 4-[(3-Chlor-4-fluor-phenyl)amino]-7-methoxy-6-{3-[6-(2-methoxy-ethyl)-2-oxo-morpholin-4-yl]-propyloxy}-chinazolin
(25)* 4-[(3-Chlor-4-fluor-phenyl)amino]-7-methoxy-6-[3-(1,9-di-oxa-2-oxo-4-aza-spiro[5.5]undecan-4-yl)-propyloxy]-chinazolin
(26)* 4-[(3-Chlor-4-fluor-phenyl)amino]-6-methoxy-7-{3-[4-(2-oxo-tetrahydrofuran-4-yl)-piperazin-1-yl]-propyloxy}-chinazolin
(27)* 4-[(3-Chlor-4-fluor-phenyl)amino]-6-methoxy-7-(3-{4-[(2-oxo-tetrahydrofuran-5-yl)methyl]-piperazin-1-yl}-propyloxy)-chinazolin
(28)* 4-[(3-Chlor-4-fluor-phenyl)amino]-6-methoxy-7-(3-{4-[(2-oxo-tetrahydrofuran-5-yl)carbonyl]-piperazin-1-yl}-propyloxy)-chinazolin
(29)* 4-[(3-Chlor-4-fluor-phenyl)amino]-6-methoxy-7-(3-{4-{2-[(2-oxo-tetrahydrofuran-3-yl)sulfanyl]-ethyl}-piperazin-1-yl}-propyloxy)-chinazolin
(30)* 4-[(3-Chlor-4-fluor-phenyl)amino]-6-methoxy-7-{3-[1-(2-oxo-tetrahydrofuran-4-yl)-piperidin-4-yl]-propyloxy}-chinazolin
(31)* 4-[(3-Chlor-4-fluor-phenyl)amino]-6-methoxy-7-[3-(3-oxo-perhydro-pyrazino[2,1-c][1,4]oxazin-8-yl)-propyloxy]-chinazolin
(32)* 4-[(3-Chlor-4-fluor-phenyl)amino]-6-methoxy-7-[3-(1-oxo-perhydro-pyrazino[2,1-c][1,4]oxazin-8-yl)-propyloxy]-chinazolin
(33)* 4-[(3-Chlor-4-fluor-phenyl)amino]-6-methoxy-7-[3-(2-oxa-3-oxo-8-aza-spiro[4.5]dec-8-yl)-propyloxy]-chinazolin
(34)* 4-[(3-Chlor-4-fluor-phenyl)amino]-6-methoxy-7-[3-(3-oxa-2-oxo-9-aza-spiro[5.5]undecan-9-yl)-propyloxy]-chinazolin
(35)* 4-[(3-Chlor-4-fluor-phenyl)amino]-6-cyclopropylmethoxy-7-[3-(3-oxa-2-oxo-9-aza-spiro[5.5]undecan-9-yl)-propyloxy]-chinazolin
(36)* 4-[(3-Chlor-4-fluor-phenyl)aminol-6-methoxy-7-[2-(3-oxa-2-oxo-9-aza-spiro[5.5]undecan-9-yl)-ethoxy]-chinazolin
(37)* 4-[(R)-(1-Phenyl-ethyl)amino]-6-methoxy-7-[2-(3-oxa-2-oxo-9-aza-spiro[5.5]undecan-9-yl)-ethoxy]-chinazolin
(38)* 4-[(3-Chlor-4-fluor-phenyl)amino]-6-methoxy-7-[3-(1,4-di-oxa-2-oxo-9-aza-spiro[5.5]undecan-9-yl)-propyloxy]-chinazolin
(39)* 4-[(3-Chlor-4-fluor-phenyl)amino]-6-methoxy-7-[3-(4-methyl-1-oxa-2-oxo-4,9-diaza-spiro[5.5]undecan-9-yl)-propyloxy]-chinazolin
(40)* 4-[(3-Chlor-4-fluor-phenyl)amino]-6-cyclopropylmethoxy-7-[3-(4-methyl-1-oxa-2-oxo-4,9-diaza-spiro[5.5]undecan-9-yl)-propyloxy]-chinazolin
(41)* 4-[(3-Chlor-4-fluor-phenyl)amino]-6-cyclopropylmethoxy-7-[2-(4-methyl-1-oxa-2-oxo-4,9-diaza-spiro[5.5]undecan-9-yl)-ethoxy]-chinazolin
(42)* 4-[(3-Chlor-4-fluor-phenyl)amino]-6-methoxy-7-{3-[4-(2-oxo-morpholin-4-yl)-piperidin-1-yl]-propyloxy}-chinazolin
(43)* 4-[(3-Chlor-4-fluor-phenyl)amino]-6-methoxy-7-{2-[4-(2-oxo-morpholin-4-yl)-piperidin-1-yl]-ethoxy}-chinazolin
(44)* 4-[(3-Chlor-4-fluor-phenyl)amino]-6-methoxy-7-{3-[4-(6-methyl-2-oxo-morpholin-4-yl)-piperidin-1-yl]-propyloxy}-chinazolin
(45)* 4-[(3-Chlor-4-fluor-phenyl)amino]-6-cyclopropylmethoxy-7-{3-[4-(6-methyl-2-oxo-morpholin-4-yl)-piperidin-1-yl]-propyloxy}-chinazolin
(46)* 4-[(3-Chlor-4-fluor-phenyl)aminol-6-methoxy-7-(3-{4-[(6-methyl-2-oxo-morpholin-4-yl)methyl]-piperidin-1-yl}-propyloxy)-chinazolin
(47)* 4-[(3-Chlor-4-fluor-phenyl)amino]-6-methoxy-7-(3-{4-[(2-oxo-tetrahydrofuran-3-yl)sulfanyl]-piperidin-1-yl}-propyloxy)-chinazolin
(48)* 4-[(3-Chlor-4-fluor-phenyl)amino]-6-methoxy-7-[3-(6-methoxymethyl-2-oxo-morpholin-4-yl)-propyloxy]-chinazolin
(49)* 4-[(3-Chlor-4-fluor-phenyl)amino]-6-methoxy-7-{3-[6-(2-methoxy-ethyl)-2-oxo-morpholin-4-yl]-propyloxy}-chinazolin
(50)* 4-[(3-Chlor-4-fluor-phenyl)amino]-6-methoxy-7-[3-(1,9-di-oxa-2-oxo-4-aza-spiro[5.5]undecan-4-yl)-propyloxy]-chinazolin
(51) 4-[(3-Chlor-4-fluor-phenyl)amino]-7-(tetrahydrofuran-3-yloxy)-6-{2-[4-(2-oxo-morpholin-4-yl)-piperidin-1-yl]-ethoxy}-chinazolin
(52) 4-[(3-Chlor-4-fluor-phenyl)amino]-7-(tetrahydropyran-4-yloxy)-6-{4-[4-(2-oxo-morpholin-4-yl)-piperidin-1-yl]-butyloxy}-chinazolin
(53) 4-[(3-Chlor-4-fluor-phenyl)amino]-7-(tetrahydrofuran-2-ylmethoxy)-6-{2-[4-(2-oxo-morpholin-4-yl)-piperidin-1-yl]-ethoxy}-chinazolin
(54) 4-[(3-Chlor-4-fluor-phenyl)amino]-7-(tetrahydropyran-4-ylmethoxy)-6-{2-[4-(2-oxo-morpholin-4-yl)-piperidin-1-yl]-ethoxy}-chinazolin
(55)* 4-[(3-Chlor-4-fluor-phenyl)amino]-6-(tetrahydrofuran-3-yloxy)-7-{2-[4-(2-oxo-morpholin-4-yl)-piperidin-1-yl]-ethoxy}-chinazolin
(56)* 4-[(3-Chlor-4-fluor-phenyl)amino]-6-(tetrahydropyran-4-yloxy)-7-{4-[4-(2-oxo-morpholin-4-yl)-piperidin-1-yl]-butyloxy}-chinazolin
(57)* 4-[(3-Chlor-4-fluor-phenyl)amino]-6-(tetrahydrofuran-2-ylmethoxy)-7-{2-[4-(2-oxo-morpholin-4-yl)-piperidin-1-yl]-ethoxy}-chinazolin
(58)* 4-[(3-Chlor-4-fluor-phenyl)amino]-6-(tetrahydropyran-4-ylmethoxy)-7-{2-[4-(2-oxo-morpholin-4-yl)-piperidin-1-yl]-ethoxy}-chinazolin

### Beispiel 7

### Dragées mit 75 mg Wirksubstanz

| 1 Dragéekern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | | |
|---|---|---|
| Kerngewicht: | 230 mg | |
| Stempel: | 9 mm, | gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 8

### Tabletten mit 100 mg Wirksubstanz

| Zusammensetzung: | |
|---|---|
| 1 Tablette enthält: | |
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.
Tablettengewicht: 220 mg
Durchmesser: 10 mm, biplan mit beidseitiger Facette und einseitiger Teilkerbe.

### Beispiel 9

### Tabletten mit 150 mg Wirksubstanz

| Zusammensetzung: | |
|---|---|
| 1 Tablette enthält: | |
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | | |
|---|---|---|
| Tablettengewicht: | 300 mg | |
| Stempel: | 10 mm, | flach |

### Beispiel 10

### Hartgelatine-Kapseln mit 150 mg Wirksubstanz

| 1 Kapsel enthält: | | |
|---|---|---|
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 11

### Suppositorien mit 150 mg Wirksubstanz

| 1 Zäpfchen enthält: | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

### Herstellung:

Nach dem Aufschmelzen der Suppositorienmasse wird der Wirkstoff darin homogen verteilt und die Schmelze in vorgekühlte Formen gegossen.

### Beispiel 12

### Suspension mit 50 mg Wirksubstanz

| 100 ml Suspension enthalten: | | |
|---|---|---|
| Wirkstoff | | 1,00 g |
| Carboxymethylcellulose-Na-Salz | | 0,10 g |
| p-Hydroxybenzoesäuremethylester | | 0,05 g |
| p-Hydroxybenzoesäurepropylester | | 0,01 g |
| Rohrzucker | | 10,00 g |
| Glycerin | | 5,00 g |
| Sorbitlösung 70%ig | | 20,00 g |
| Aroma | | 0,30 g |
| Wasser dest. | ad | 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

### Beispiel 13

### Ampullen mit 10 mg Wirksubstanz

| Zusammensetzung: | | |
|---|---|---|
| Wirkstoff | | 10,0 mg |
| 0,01N Salzsäure s.q. | | |
| Aqua bidest | ad | 2,0 ml |

### Herstellung:

Die Wirksubstanz wird in der erforderlichen Menge 0,01N HCl gelöst, mit Kochsalz isotonisch gestellt, sterilfiltriert und in 2 ml Ampullen abgefüllt.

### Beispiel 14

### Ampullen mit 50 mg Wirksubstanz

| Zusammensetzung: | | |
|---|---|---|
| Wirkstoff | | 50,0 mg |
| 0,01N Salzsäure s.q. | | |
| Aqua bidest | ad | 10,0 ml |

### Herstellung:

Die Wirksubstanz wird in der erforderlichen Menge 0,01N HCl gelöst, mit Kochsalz isotonisch gestellt, sterilfiltriert und in 10 ml Ampullen abgefüllt.

### Beispiel 15

### Kapseln zur Pulverinhalation mit 5 mg Wirksubstanz

| 1 Kapsel enthält: | |
|---|---|
| Wirksubstanz | 5,0 mg |
| Lactose für Inhalationszwecke | 15,0 mg |
| | 20,0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.

| | |
|---|---|
| Kapselgewicht: | 70,0 mg |
| Kapselgröße: | 3 |

### Beispiel 16

### Inhalationslösung für Handvernebler met 2,5 mg Wirksubstanz

| 1 Hub enthält: | |
|---|---|
| Wirksubstanz | 2,500 mg |
| Benzalkoniumchlorid | 0,001 mg |
| 1N-Salzsäure q.s. | |
| Ethanol/Wasser (50/50) | ad 15,000 mg |

### Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.
Füllmasse des Behälters: 4,5 g

## Patentansprüche

1. Bicyclische Heterocyclen der allgemeinen Formel 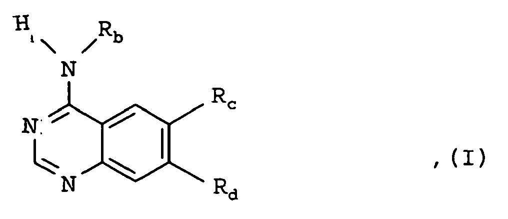 in der
R_{b} eine 1-Phenylethyl-, 3-Methylphenyl-, 3-Chlorphenyl-, 3-Bromphenyl- oder 3-Chlor-4-fluorphenylgruppe,
R_{c} eine -A-B Gruppe, in der
A eine -OCH₂CH₂-, -OCH₂CH₂CH₂- oder -OCH₂CH₂CH₂CH₂- Gruppe, wobei der Alkylenteil jeweils mit dem Rest B verknüpft ist, und
B eine Piperidinogruppe, die in 4-Stellung durch eine 2-Oxo-morpholino- oder 2-Oxo-morpholinomethylgruppe substituiert ist, wobei der 2-Oxo-morpholinoteil jeweils durch eine oder zwei Methylgruppen substituiert sein kann, darstellen,
und R_{d} eine Methoxy-, Cyclopropylmethoxy-, Tetrahydrofuran-3-yloxy-, Tetrahydropyran-3-yloxy-, Tetrahydropyran-4-yloxy-, Tetrahydrofuranylmethoxy oder Tetrahydropyranylmethoxygruppe bedeuten,
deren Tautomere, Stereoisomere und deren Salze.

2. Folgende Verbindungen der allgemeinen Formel I gemäß Anspruch 1:
(1) 4-[(3-Chlor-4-fluor-phenyl)amino]-6-{2-[4-(2-oxo-morpholin-4-yl)-piperidin-1-yl]-ethoxy}-7-((*R*)-tetrahydrofuran-3-yloxy)-chinazolin,
(2) 4-[(3-Chlor-4-fluor-phenyl)amino]-6-{2-[4-(2-oxo-morpholin-4-yl)-piperidin-1-yl]-ethoxy}-7-methoxy-chinazolin,
(3) 4-[(3-Chlor-4-fluor-phenyl)amino]-7-methoxy-6-{3-[4-(2-oxo-morpholin-4-yl)-piperidin-1-yl]-propyloxy}-chinazolin,
(4) 4-[(3-Chlor-4-fluor-phenyl)amino]-7-methoxy-6-{3-[4-(6-methyl-2-oxo-morpholin-4-yl)-piperidin-1-yl]-propyloxy}-chinazolin,
(5) 4-[(3-Chlor-4-fluor-phenyl)amino]-7-cyclopropylmethoxy-6-{3-[4-(6-methyl-2-oxo-morpholin-4-yl)-piperidin-1-yl]-propyloxy}-chinazolin,
(6) 4-[(3-Chlor-4-fluor-phenyl)amino]-7-methoxy-6-(3-{4-[(6-methyl-2-oxo-morpholin-4-yl)methyl]-piperidin-1-yl}-propyloxy)-chinazolin,
(7) 4-[(3-Chlor-4-fluor-phenyl)amino]-7-(tetrahydrofuran-3-yloxy)-6-{2-[4-(2-oxo-morpholin-4-yl)-piperidin-1-yl]-ethoxy}-chinazolin,
(8) 4-[(3-Chlor-4-fluor-phenyl)amino]-7-(tetrahydropyran-4-yloxy)-6-{4-[4-(2-oxo-morpholin-4-yl)-piperidin-1-yl]-butyloxy}-chinazolin,
(9) 4-[(3-Chlor-4-fluor-phenyl)amino]-7-(tetrahydrofuran-2-ylmethoxy)-6-{2-[4-(2-oxo-morpholin-4-yl)-piperidin-1-yl]-ethoxy}-chinazolin und
(10) 4-[(3-Chlor-4-fluor-phenyl)amino]-7-(tetrahydropyran-4-ylmethoxy)-6-{2-[4-(2-oxo-morpholin-4-yl)-piperidin-1-yl]-ethoxy}-chinazolin,
deren Tautomere, Stereoisomere und deren Salze.

3. Physiologisch verträgliche Salze der Verbindungen nach einem der Ansprüche 1 oder 2 mit anorganischen oder organischen Säuren oder Basen.

4. Arzneimittel, enthaltend eine Verbindung nach einem der Ansprüche 1 oder 2 oder ein physiologisch verträgliches Salz gemäß Anspruch 3 neben gegebenenfalls einem oder mehreren inerten Trägerstoffen und/oder Verdünnungsmitteln.

5. Verwendung einer Verbindung nach mindestens einem der Ansprüche 1 bis 3 zur Herstellung eines Arzneimittels, das zur Behandlung von benignen oder malignen Tumoren, zur Vorbeugung und Behandlung von Erkrankungen der Atemwege und der Lunge, zur Behandlung von Polypen, von Erkrankungen des Magen-Darm-Traktes, der Gallengänge und -blase sowie der Niere und der Haut geeignet ist.

6. Verfahren zur Herstellung eines Arzneimittels gemäß Anspruch 4, **dadurch gekennzeichnet, daß** auf nichtchemischem Wege eine Verbindung nach mindestens einem der Ansprüche 1 bis 3 in einen oder mehrere inerte Trägerstoffe und/oder Verdünnungsmittel eingearbeitet wird.

## Claims

1. Bicyclic heterocycles of general formula 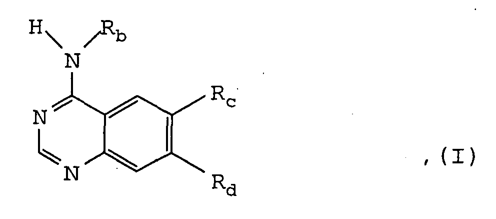 wherein
R_{b} denotes a 1-phenylethyl, 3-methylphenyl, 3-chlorophenyl, 3-bromophenyl or 3-chloro-4-fluorophenyl group,
R_{c} denotes an -A-B group wherein
A denotes a -OCH₂CH₂, -OCH₂CH₂CH₂ or -OCH₂CH₂CH₂CH₂ group, wherein the alkylene moiety in each case is linked to the group B, and
B denotes a piperidino group which is substituted in the 4 position by a 2-oxo-morpholino or 2-oxo-morpholinomethyl group, where the 2-oxo-morpholino moiety may be substituted in each case by one or two methyl groups,
and R_{d} represents a methoxy, cyclopropylmethoxy, tetrahydrofuran-3-yloxy, tetrahydropyran-3-yloxy, tetrahydropyran-4-yloxy, tetrahydrofuranylmethoxy or tetrahydropyranylmethoxy group,
the tautomers, stereoisomers and the salts thereof.

2. The following compounds of general formula I according to claim 1:
(1) 4-[(3-chloro-4-fluoro-phenyl)amino]-6-{2-[4-(2-oxo-morpholin-4-yl)-piperidin-1-yl]-ethoxy}-7-((*R*)-tetrahydrofuran-3-yloxy)-quinazoline
(2) 4-[(3-chloro-4-fluorophenyl)amino]-6-{2-[4-(2-oxo-morpholin-4-yl)-piperidin-1-yl]-ethoxy}-7-methoxy-quinazoline,
(3) 4-[(3-chloro-4-fluorophenyl)amino]-7-methoxy-6-{3-[4-(2-oxo-morpholin-4-yl)-piperidin-1-yl]-propyloxy}-quinazoline,
(4) 4-[(3-chloro-4-fluorophenyl)amino]-7-methoxy-6-{3-[4-(6-methyl-2-oxo-morpholin-4-yl)-piperidin-1-yl]-propyloxy}-quinazoline,
(5) 4-[(3-chloro-4-fluorophenyl)amino]-7-cyclopropylmethoxy-6-{3-[4-(6-methyl-2-oxo-morpholin-4-yl)-piperidin-1-yl]-propyloxy}-quinazoline,
(6) 4-[(3-chloro-4-fluorophenyl)amino]-7-methoxy-6-(3-{4-[(6-methyl-2-oxo-morpholin-4-yl)methyl]-piperidin-1-yl}-propyloxy)-quinazoline,
(7) 4-[(3-chloro-4-fluorophenyl)amino]-7-(tetrahydrofuran-3-yloxy)-6-{2-[4-(2-oxo-morpholin-4-yl)-piperidin-1-yl]-ethoxy}-quinazoline,
(8) 4-[(3-chloro-4-fluorophenyl)amino]-7-(tetrahydropyran-4-yloxy)-6-{4-[4-(2-oxo-morpholin-4-yl)-piperidin-1-yl]-butyloxy}-quinazoline,
(9) 4-[(3-chloro-4-fluorophenyl)amino]-7-(tetrahydrofuran-2-ylmethoxy)-6-{2-[4-(2-oxo-morpholin-4-yl)-piperidin-1-yl]-ethoxy}-quinazoline and
(10) 4-[(3-chloro-4-fluorophenyl)amino]-7-(tetrahydropyran-4-ylmethoxy)-6-{2-[4-(2-oxo-morpholin-4-yl)-piperidin-1-yl]-ethoxy}-quinazoline,
the tautomers, stereoisomers and the salts thereof.

3. Physiologically acceptable salts of the compounds according to one of claims 1 or 2 with inorganic or organic acids or bases.

4. Pharmaceutical compositions containing a compound according to one of claims 1 or 2 or a physiologically acceptable salt according to claim 3 optionally together with one or more inert carriers and/or diluents.

5. Use of a compound according to at least one of claims 1 to 3 for preparing a pharmaceutical composition which is suitable for treating benign or malignant tumours, for preventing and treating diseases of the airways and lungs, for treating polyps, diseases of the gastrointestinal tract, the bile duct and gall bladder as well as the kidneys and skin.

6. Process for preparing a pharmaceutical composition according to claim 4, **characterised in that** a compound according to at least one of claims 1 to 3 is incorporated in one or more inert carriers and/or diluents by a non-chemical method.

## Revendications

1. Hétérocycles bicycliques de formule générale 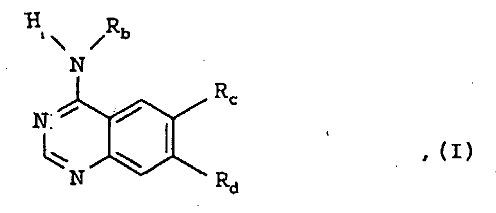 où
R_{b} représente un groupe 1-phényléthyle, 3-méthylphényle, 3-chlorophényle, 3-bromophényle ou 3-chloro-4-fluorophényle,
R_{c} représente un groupe -A-B où
A représente un groupe -OCH₂CH₂-, -OCH₂CH₂CH₂- ou -OCH₂CH₂CH₂CH₂-, où la partie alkylène est liée à chaque fois avec le groupement B,
et
B représente un groupe pipéridino, qui est substitué en position 4 par un groupe 2-oxo-morpholino ou 2-oxo-morpholinométhyle, où la partie 2-oxo-morpholino peut être substituée à chaque fois par un ou deux groupes méthyle,
et R_{d} représente un groupe méthoxy, cyclopropylméthoxy, tétrahydrofuran-3-yloxy, tétrahydropyran-3-yloxy, tétrahydropyran-4-yloxy, tétrahydrofuranylméthoxy ou tétrahydropyranylméthoxy,
leurs tautomères, leurs stéréoisomères et leurs sels.

2. Composés de formule générale 1 selon la revendication 1 suivants :
(1) 4-[(3-chloro-4-fluoro-phényl)amino]-6- {2-[4-(2-oxo-morpholin-4-yl)-pipéridin-1-yl]-éthoxy}-7-((*R*)-tétrahydrofuran-3-yloxy)-quinazoline,
(2) 4-[(3-chloro-4-fluoro-phényl)amino]-6-{2-[4-(2-oxo-morpholin-4-yl)-pipéridin-1-yl]-éthoxy}-7-méthoxy-quinazoline,
(3) 4-[(3-chloro-4-fluoro-phényl)amino]-7-méthoxy-6-{3-[4-(2-oxo-morpholin-4-yl)-pipéridin-1-yl]-propyloxy}-quinazoline,
(4) 4-[(3-chloro-4-fluoro-phényl)amino]-7-méthoxy-6-{3-[4-(6-méthyl-2-oxo-morpholin-4-yl)-pipéridin-1-yl]-propyloxy}-quinazoline,
(5) 4-[(3-chloro-4-fluoro-phényl)amino]-7-cyclopropylméthoxy-6-{3-[4-(6-méthyl-2-oxo-morpholin-4-yl)-pipéridin-1yl]-propyloxy}-quinazoline,
(6) 4-[(3-chloro-4-fluoro-phényl)amino]-7-méthoxy-6-(3-{4-[6-méthyl-2-oxo-morpholin-4-yl)méthyl]-pipéridin-1-yl}-propyloxy)-quinazoline,
(7) 4-[(3-chloro-4-fluoro-phényl)amino]-7-(tétrahydrofuran-3-yloxy)-6-{2-[4-(2-oxo-morpholin-4-yl)-pipéridin-1-yl]-éthoxy}-quinazoline,
(8) 4-[(3-chloro-4-fluoro-phényl)amino]-7-(tétrahydropyran-4-yloxy)-6-{4-[4-(2-oxo-morpholin-4-yl)-pipéridin-1-yl]-butyloxy}-quinazoline,
(9) 4-[(3-chloro-4-fluoro-phényl)amino]-7-(tétrahydrofuran-2-ylméthoxy)-6-{2-[4-(2-oxo-morpholin-4-yl)-pipéridin-1-yl]-éthoxy}-quinazoline et
(10) 4-[(3-chloro-4-fluoro-phényl)amino]-7-(tétrahydropyran-4-ylméthoxy)-6-{2-[4-(2-oxo-morpholin-4-yl)-pipéridin-1-yl]-éthoxy}-quinazoline,
leurs tautomères, leurs stéréoisomères et leurs sels.

3. Sels physiologiquement acceptables des composés selon l'une des revendications 1 ou 2 avec des acides ou des bases inorganiques ou organiques.

4. Médicament contenant un composé selon l'une des revendications 1 ou 2 ou un sel physiologiquement acceptable selon la revendication 3 outre éventuellement un ou plusieurs supports et/ou diluants inertes.

5. Utilisation d'un composé selon au moins l'une des revendications 1 à 3 pour la production d'un médicament qui convient pour le traitement des tumeurs bénignes ou malignes, pour la prévention et le traitement des maladies des voies respiratoires et du poumon, pour le traitement des polypes, des maladies des voies gastro-intestinales, des voies biliaires et de la vésicule biliaire ainsi que du rein et de la peau.

6. Procédé de production d'un médicament selon la revendication 4 **caractérisé en ce que**, par voie non chimique, un composé selon au moins l'une des revendications 1 à 3 est incorporé dans un ou plusieurs supports et/ou diluants inertes.
